# EUROPEAN PATENT SPECIFICATION

(11) **EP 3 576 827 B1**
(45) Date of publication and mention of the grant of the patent: **14.09.2022**
(21) Application number: 18761150.4
(22) Date of filing: 28.02.2018
(51) Int. Cl.: A61M 25/00, A61M 27/00, A61M 1/00

(54) **MEDICAL INSERT**
MEDIZINISCHER EINSATZ
INSERT MÉDICAL

(30) Priority: 28.02.2017 AU 2017900688
(43) Date of publication of application: 11.12.2019
(73) Proprietor: Koefman, Alexander, Hawthorn, Queensland QLD 4171 (AU)
(72) Inventor: Koefman, Alexander, Hawthorn, Queensland QLD 4171 (AU)
(74) Representative: Schneiter, Sorin
(86) International application number: PCT/AU2018/050182
(87) International publication number: WO 2018/157206

(56) References cited:
- EP-A1- 0 583 070
- US-A- 3 516 410
- US-A- 3 516 410
- US-A- 3 626 950
- US-A- 5 304 155
- US-A1- 2005 043 703
- US-A1- 2009 054 825
- US-A1- 2014 207 045
- US-A1- 2016 354 577
- US-B1- 9 364 647
- US-B1- 9 364 647

## Description

### Field of invention

This invention relates to a subcutaneously insertable medical device and, in particular, to a catheter having coverings to inhibit ingress of brain tissue.

### Background to the invention

Cerebrospinal fluid ('CSF') is continually produced deep within the brain and circulates to the surface of the brain via a series of channels and cisterns. From here, it is reabsorbed into the veins that simultaneously drain blood from the brain. Disorders of CSF arise when this circulatory network (the ventricular system) in the brain becomes blocked (which could occur for one of many reasons), or the reabsorption of CSF into the veins is impeded. This creates a situation of increasing CSF volume within the brain, and a subsequent increase in pressure on the brain. This increased pressure can be life threatening.

Treatment for the majority of such disorders is drainage of CSF from the brain. This is achieved by utilising a shunt system, which involves inserting a catheter into one of the ventricles within the brain, and redirecting the flow of CSF externally, thus relieving the increased pressure. The ventricular catheter is composed of a hollow silicone tube with perforations to the distal end, allowing outflow of CSF via the perforations into the tube. The catheter may either be connected to a drainage system that remains external to the patient, or may be fully internalised with the terminal tubing connection being positioned in another body cavity, commonly the abdomen, where the CSF is reabsorbed.

The two main complications arising from this tubing system are infection and blockage. Infection rates have significantly reduced with the advancement of modern surgical techniques, and with the use of pre-operative intravenous antibiotics and antibiotic impregnated catheters. Unfortunately, similar advances have not been realised with regard to shunt blockages. When the shunt system blocks, often the ventricular catheter needs to be removed and replaced with a new one. The cost of a new, replacement ventricular catheter is significant. On occasion, the shunt valve connecting the ventricular catheter to the distal draining catheter needs to be replaced. The price of these valves is an order of magnitude greater.

CSF disorders are very common and, therefore, operations to insert shunt systems are also very common. Indeed, blockage of the shunt is the most common reason for shunt revision surgery and historically, choroid plexus is implicated as the main culprit. Choroid plexus is the tissue that produces CSF, and lines the ventricular system within the brain. It has been theorised that choroid plexus grows into the ventricular catheter perforations, thus occluding the catheter. However, clinical experience suggests that it is instead more likely the brain tissue itself that causes the majority of shunt blockages, and this occurs during insertion of the catheter. Any brain tissue within the shunt system can block the ventricular catheter lumen, the shunt valve or the distal catheter. The brain tissue is somewhat collected by the distal perforations of the ventricular catheter during insertion, due to the 'cheese grater' effect of the perforations as the catheter passes through brain tissue to reach the ventricles inside the brain. With each revision surgery a patient undergoes to replace a blocked shunt, the risk of shunt infection increases, hence it is imperative from both an economic and clinical standpoint to reduce the incidence of shunt blockage.

US patent no. 9,364,647 addresses shunt catheter systems and describes an open-ended ventricular catheter with drainage perforations and safety flap valves, a reservoir with inline filter, and a peritoneal catheter. It observes that *'Conventional stylets typically are square-ended (and would damage tissue during insertion)'.* It is not suggested that the catheter should or may have a similar shape or square cross-section.

Publication WO2013119425, an international patent application, discloses a stylet of non-round cylindrical cross-section for use in blocking ingress of tissue into a catheter from the exterior. It does not discuss flexible flap-like external or internal closures, let alone the possibility of combining such devices with the stylet. Paragraph [0066] thereof discusses embodiments shown in Figure 17 thereof. This figure illustrates an intricate device, having prongs extending from a shaft inserted into the catheter so that flexible covers are pushed out of the perforations in the catheter to cover them in an umbrella-like manner. This still does not suggest attaching the covers to a flat external surface of the catheter itself.

Hakim, in US 3,516,410, discloses a catheter of rounded axial profile having externally attached flaps for covering the holes in the perforation as it passes through brain tissue. The major drawback inherent to this device was that the flap design and material did not allow sufficient flexibility for the flaps to be pushed flat against the ventricular catheter, which not only failed to entirely conceal the perforations, but also caused unnecessary trauma to the brain during insertion or removal by creating a track diameter that significantly exceeded the diameter of the ventricular catheter alone. The ingress of tissue fragments risks blockages in the catheter, or downstream in the patient of other apparatus for treating extracted fluids.

US published patent application 2009/0054825 (Melsheimer et al) discloses a winged catheter assembly, housing dual lumens in a single tube of circular cross-section that changes to a flattened profile at a distal portion leading up to the distal end. The distal flattened profile and surface are said to contribute to ease of insertion into tissue and body compartments. The inventors were not concerned with the mode of covering drainage apertures in brain tissue.

The *Melsheimer* design is considered unsuitable for insertion into brain tissue, as it would result in potential unnecessary damage to the brain tissue during insertion and withdrawal as the flaps covering the perforations close circumferentially. Such a design would result in a "cheese grater" effect, whereby tissue fragments are scraped away from surrounding tissue. This would encourage brain tissue to enter the catheter lumen, which is a complication best avoided.

Furthermore, the Melsheimer design would not allow for efficient drainage of brain fluid once inserted, due to the unique structure of the wall of the cavities within the brain. These cavities are small, and the lining firm. The Melsheimer design would likely result in occlusion of the flaps if the catheter were to rest against the wall of the brain cavity. Further, high pressure within the brain cavities would likely occlude the perforation flaps. The disclosed prior catheter is therefore not suitable for use in brain tissue. This is because brain tissue is uniquely different to all other tissue types, in particular blood vessels, for which Melsheimer's device is designed. Blood vessels have a firm wall and in this context the cheese grater effect is not a relevant factor.

A need therefore exists for a catheter that is suitable for insertion and withdrawal without the cheese grater effect, while still being effective to provide continuous drainage of fluid from the brain cavity.

### Objects of the invention

It is an object of this invention to address the shortcomings of the prior art and, in doing so, to provide a substantially sealing cover for the radially opening perforations of a catheter when passing through brain tissue.

A further object of the invention is to provide a device, the use of which will reduce risk and incidence of shunt blockage.

The preceding discussion of the background to the invention is intended to facilitate an understanding of the present invention. However, it should be appreciated that the discussion is not an acknowledgement or admission that any of the material referred to was part of the common general knowledge in Australia or elsewhere as at the priority date of the present application.

Further, and unless the context clearly requires otherwise, throughout the description and the claims, the words 'comprise', 'comprising', and the like are to be construed in an inclusive sense - that is to say, in the sense of "including, but not being limited to" - as opposed to an exclusive or exhaustive sense - meaning "including this and nothing else".

### Summary of invention

According to the invention, there is provided a catheter for removing cerebrospinal fluid from the brain, the catheter comprising an elongate body having an internal lumen and an exterior surface, a portion of which is substantially planar, a perforation that extends through the planar exterior surface, providing fluid communication between the lumen and said surface portion, and a movable covering operatively connected to the body to occlude said perforation in response to relative movement between the catheter and tissue during insertion into and withdrawal from said tissue.

In an embodiment of the invention, the covering stands proud from the body when the catheter is operatively located, thereby uncovering the perforation, wherein the covering is preferably foldable to occlude the perforation and/or wherein the covering is preferably foldable in alternative opposite directions.

According to another embodiment of the invention, the perforation is associated with a further, second covering. The first covering is located upstream and the second downstream of the perforation when the catheter is undergoing insertion into receiving tissue.

Still further, in yet another embodiment, the covering comprises a flap.

The flap preferably comprises a fold line along which the flap is foldable to occlude the perforation, wherein the flap is preferably resiliently flexible.

In a further embodiment, the covering and body are integral with each other. The catheter may be manufactured, for example, by way of a moulding process or by three-dimensional printing.

Further, according to yet another embodiment of the invention, the lumen has a profile corresponding in shape to the axial profile of the body.

In another embodiment, the body comprises interconnected distal and proximal portions, having different respective axial profiles.

Further preferably, the distal portion includes the perforation.

Still further, the distal portion preferably has a generally rectangular axial profile. For example, the profile is of generally square configuration.

The flap may for example have a proximal edge disposed against the surface portion.

The catheter is generally characterized in having at least one flat external surface through which a plurality of perforations extend.

According to a second aspect of the disclosure, not forming part of the invention, there is provided a method of removing excess cerebrospinal fluid from a patient, the method comprising the steps of:
a. providing a catheter having an elongate body with an axial lumen and a substantially planar exterior surface portion, through which a perforation extends to provide fluid communication between the lumen and the exterior of the catheter, and a covering moveable into an occluding configuration when under tissue pressure during movement of the catheter in an axial direction within tissue, thereby to occlude the perforation, and into a perforation-exposing configuration when said movement ceases;
b. advancing the catheter into brain tissue of the recipient, thereby causing the perforation to be occluded,
c. allowing the catheter to be stationary in relation to the tissue, whereby the perforation is exposed for allowing CSF to enter the lumen through the perforation, and
d. withdrawing the catheter from the tissue, whereby the action of withdrawing causes occlusion of the perforation.

In a preferred form, the method includes providing a catheter having one or more of the features described in the preceding paragraphs. Preferably, the covering is foldable to occlude the perforation. Further preferably, the covering is foldable in opposite directions. In a preferred example, the perforation is associated with two coverings.

In an example, the method includes forming the catheter with the covering integral with the body.

The method preferably further includes providing the catheter having a distal portion in which the perforation is located.

In a preferred form of the disclosure, the catheter comprises a distal portion having a proximal portion having a different axial profile than the distal portion.

In a still further preferred form of the disclosure, the axial profile of the distal portion is rectangular. Preferably, the lumen has a rectangular profile.

### Brief description of drawings

In order that the invention may be readily understood, and put into practical effect, reference will now be made to the accompanying figures. Thus:
Figure 1 shows in schematic form a cross-sectional diagram of a catheter in a preferred embodiment of this invention in three differing phases of use.
Figure 2 is a schematic diagram of the catheter in a second embodiment of the invention.
Figure 3 is a schematic axial sectional (a) and (b) views of a further embodiment of the catheter of the invention.
Figure 4 shows the profile of an alternative example of the disclosure, (a) in default extended flap configuration and (b) in motion, with the flaps folded to flat.

### Detailed description of an embodiment of the invention

The present invention is found to significantly reduce the incidence of shunt blockages by preventing the 'cheese grater' effect caused by ventricular catheter perforations during insertion and/or removal of a catheter from brain or other tissue. The reduction is achieved by providing a covering for occluding each perforation during insertion and withdrawal of the catheter from the patient's tissue.

In a preferred embodiment, each covering is provided in the form of a flexible flap associated with at least one perforation that extends through the catheter wall.

Catheters for brain fluid drainage during clinical procedures at some point all need to be removed. Placing the flaps on a planar surface of the catheter is found to help prevent the flaps from distorting during closure (for example dog ear distortion) in either the forward or reverse direction, that is during both insertion and removal. The planar surface and radial flaps that are raiseable to about 90 degrees to the planar surface are found to function for insertion into and removal from brain tissue without having a cheese grater effect in either direction. Hence damage to adjacent brain is avoided when moved in either direction.

The flap is preferably integrally formed with the catheter body. Manufacture may be by means of a moulding process known in the art, injection moulding being but one example. The catheter may also be manufactured by way of a three-dimensional printing process. The catheter may be made from known substances of medical grade, non-limiting examples including silicone, polyurethane, polyethylene, polypropylene, latex and the like.

The covering flaps are therefore formed so that they stand proud of the catheter body in their default state, but are of a thickness that enables them to flap forward or backward according to the direction of travel of the catheter through brain tissue. When the catheter is operatively located in position within the tissue, the flaps are of sufficient resilience to return to their raised posture, extending substantially perpendicularly to the surface through which the respective relevant perforation passes. In raised position, they expose the perforations to establish fluid communication between the catheter lumen and the exterior.

The flaps readily collapse forward or backward under the influence of adjacent brain tissue pressure when the catheter is moved in relation to the tissue, depending on whether the catheter is being inserted or removed. These flaps therefore conceal each perforation and render the perforations inaccessible to adjacent brain tissue while the catheter is being moved. Once positioned within the ventricular network, the flaps promptly return to their resting generally perpendicular position, thus uncovering the perforations again and allowing CSF to enter the catheter and drain through the shunt system.

The design of the present device allows for the flaps to be folded substantially flush with the exterior surface of the catheter, thereby creating minimal, if any, additional trauma to the brain tissue, whilst completely covering the catheter perforations during insertion or removal. The flush folding is accomplished by providing the distal end part of the catheter containing the perforations with a profile that exhibits at least one flat outer surface. The flaps therefore have a flat base and do not 'dog ear' when collapsing forward or backward, as they would if the catheter was rounded in cross-sectional profile. In a preferred example, the catheter has at least two planar surface portions. The number of planar facets is influenced by the number of perforations and their size. By way of non-limiting examples, the profile may be square, rectangular, triangular, pentagonal and hexagonal, etc.

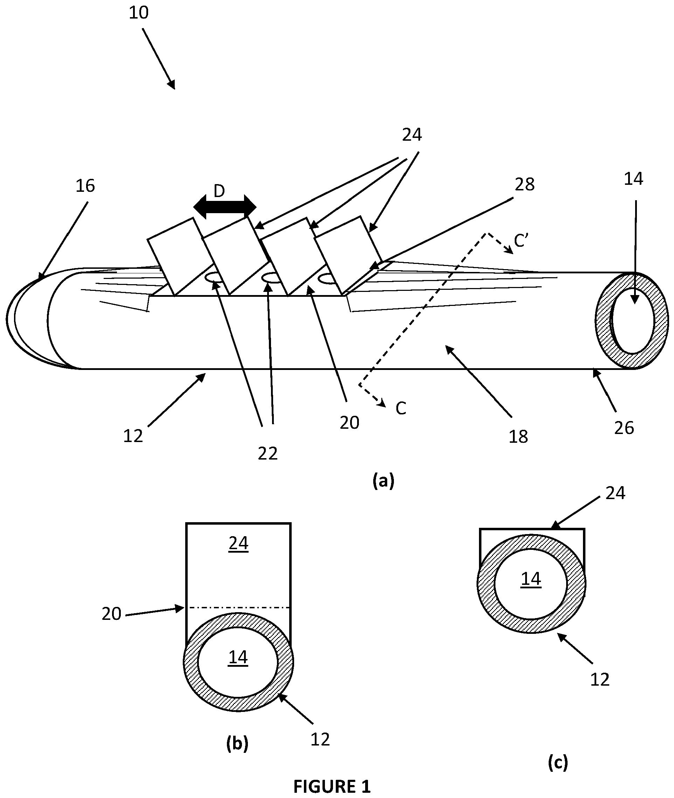

Referring to Figure 1, there is shown, in schematic perspective cutaway view, the distal portion of a ventricular catheter of the invention 10. The catheter has a hollow body 12 defining an axial lumen 14. The distal end 16 of the catheter is closed. The body has an exterior surface 18 with a flat, generally planar portion 20. Extending into portion 20 are a series of perforations 22, which provide fluid communication between lumen 14 and the exterior of the body.

Flaps 24 are positioned both proximally and distally relative to each perforation 22, such that every perforation is flanked by two such flaps. These are operable, when the catheter is moved in tissue, to conceal the respective perforations, whether the catheter is being inserted or removed.

Importantly, the perforations must be covered, not only during removal of the catheter, but also on insertion, to avoid occult brain tissue fragments remaining in the catheter after it has been flushed, as it is common for multiple attempts to cannulate the ventricle to be made with the same catheter during surgery. It is extremely difficult to remove all brain tissue fragments from a catheter that has been removed due to an unsuccessful attempt at placing it within the ventricle.

Each of the flaps is adapted to be moveable axially in both directions, as indicated by directional arrow D, allowing it to be flattened against outer surface portion 20 to cover and conceal the perforation that lies immediately behind it. In Figure 1(b) and (c), the catheter is shown in axial cross section taken along the line C-C' in Figure 1(a). In (b), flap 24 is shown in its default raised position when stationary, and in (c) it is shown flattened, as occurs during the action of insertion or removal.

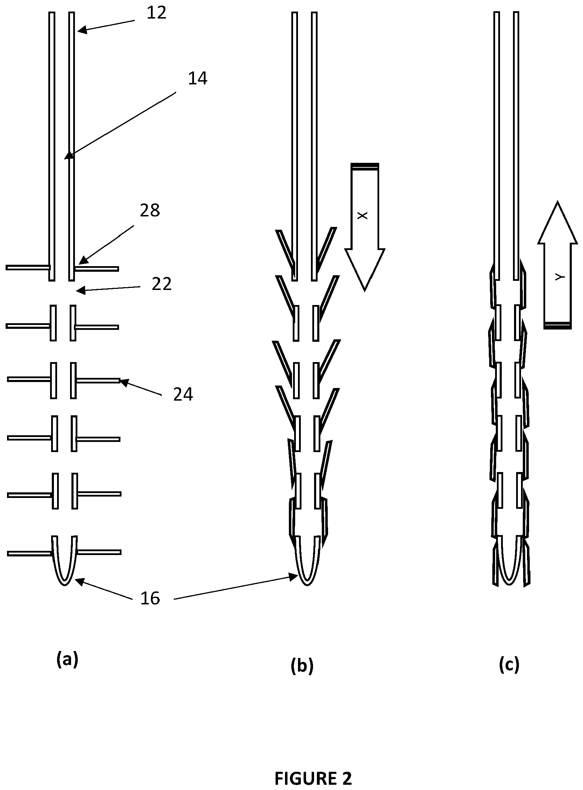

The relative positions of the flaps are further illustrated in the alternative embodiment shown in Figure 2, in which like parts from Figure 1 have like numbers. This embodiment has paired flaps extending from diametrically opposed radial positions.

In the embodiment illustrated in Figure 2(a), the flaps are shown in perpendicular extended configuration, exposing the perforations 22 for enabling flow of CSF through them. This configuration occurs both when the catheter is waiting to be inserted and when the catheter has been inserted to its operative location in the brain tissue and is stationary relative thereto.

In Figure 2(b), the flaps are shown in their general positions when the catheter is in the process of being inserted and there is relative motion between the catheter and the receiving brain tissue. Here, they are generally flattened back opposite to the direction of movement on insertion. For illustrative purposes they are shown both fully and partially flattened. The most distal, leading flaps are shown fully flattened with those following not yet fully collapsed against the external surface. However, they will become fully flattened as the surrounding tissue exerts increasing pressure with the advancing movement.

The configuration in Figure 2(c) occurs when the catheter is in the process of being withdrawn. Again, the flaps are flattened against the surface of the catheter body, but this time they are folded back in the opposite direction, to be aligned with the direction of withdrawal.

A rigid or semi-rigid stylet (not shown) can be inserted into the lumen to aid insertion into body tissue - and brain tissue in particular - without compromising the forward or reverse motion of the flaps.

The proximal portion 26 of the catheter in this embodiment is round in cross section. This is to facilitate connection of the ventricular catheter to the shunt valve or distal draining catheter (not shown), and to prevent occlusion of the catheter as it traverses the bony insertion site.

Reference is made again to Figure 1(b) and (c), where the distal catheter portion has a flat surface portion 20 when viewed in cross-section, with the flap base 28 having an edge shaped to correspond with the axial profile of the catheter exterior surface. It is found that when the flap base is positioned about a rounded cross-section, it tends to 'dog ear' and fail to completely occlude the perforations and prevent shunt blockage.

During insertion of the ventricular catheter, the soft flexible flaps collapse easily to fully occlude the preceding perforation. They are compressed due to the pressure of the adjacent brain tissue. During catheter removal, the flaps collapse in the opposite direction to once again fully occlude the preceding perforation.

The inventor's personal experience demonstrates that brain tissue will inevitably be forced into the perforations of a ventricular catheter during insertion and removal. This is particularly evident after an initial unsuccessful attempt to cannulate the ventricle, requiring the catheter to be removed before a second attempt is made. However, before a second attempt can be made, the catheter must be flushed to remove any brain tissue that has entered the perforations and hence the catheter lumen during the unsuccessful attempt. In practice, it is not logistically feasible to remove every fragment of brain tissue that enters the catheter, and it is theorised that following successful insertion of the shunt system, these remaining fragments eventually follow the flow of CSF and either block the catheter distally (this is often directly observed as the external shunt is often transparent and such fragments are clearly visible) or block the complex shunt valve mechanism. It is extremely costly to replace components of a shunt, and of relatively high risk to the patient. The present invention, it is expected, will significantly reduce medical costs and improve patient safety, particularly in light of the worldwide frequency of shunt operations.

The present invention requires a cannula tip for introduction through the brain tissue into the brain cavity, but has a configuration of flaps and apertures that allows the brain cavity to be cannulated without blockage of the catheter occurring. This is a situation unique to the brain. The brain has a soft consistency and easily enters perforations with small pressures, unlike all other bodily tissues. The proposed invention takes this into account and, unlike prior inventions, identifies a configuration that prevents this from occurring, while at the same time preventing damage to normal adjacent brain tissue.

In the above embodiments, each flap covers one perforation at a time. However, it is considered feasible in other embodiments to provide for a single flap to cover two or more perforations, depending on spacing and flap flexibility, the latter arising from factors such as materials of construction.

It will be appreciated that the flap does not extend radially around the catheter body, but is instead adapted to extend for the width of the perforation, allowing for an additional margin on each side. Preferably each margin should be of width in the range of about 3% to 75% of the perforation width, more preferably in the range from 15% to 30% thereof.

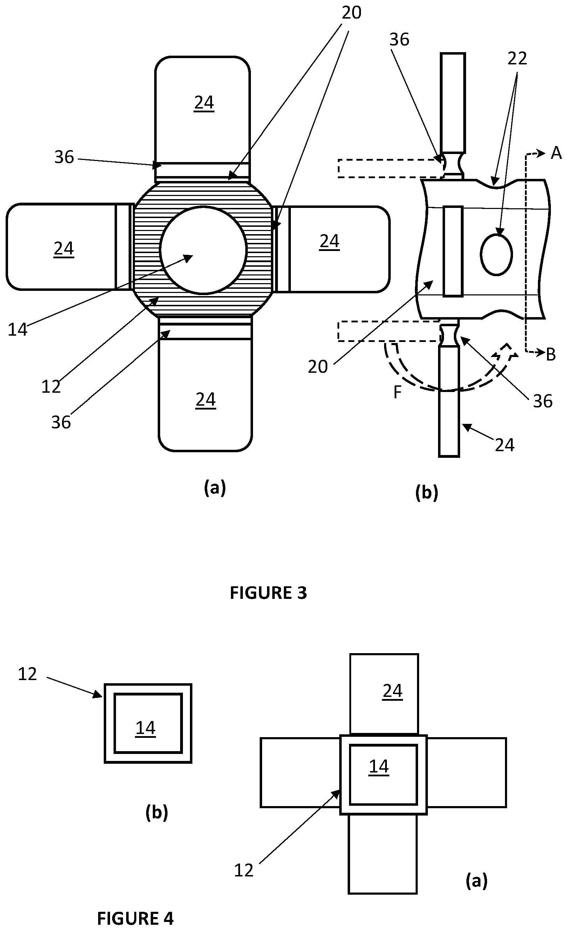

By providing for the flap to have a straight bottom edge at which it connects with a planar surface portion of the catheter body, an inherent fold line is provided, at which the flap is able to oscillate back and forth according to the direction of travel of the catheter through tissue. This is illustrated in Figure 3, in which the fold line is defined by a strip 36 of thinner material than the flap. Like parts bear the same numbering as in previous figures, with Figure 3(a) being an axial section of the catheter, taken along line A-B in Figure 3(b), which provides a partially cut-away side view. Each flap 24 is able to be folded along strip 36 to assume a flattened position, substantially as shown by the broken lined strips pointed out by directional arrow F. The strip is resilient to allow the flap to assume its default erect posture when relative movement with the surrounding tissue ceases.

Figure 4 illustrates an example in which the distal portion of the catheter has a generally square profile and in which the body 12 is in the form of a square cylindrical tube, in which lumen 14 is of square profile, and from which flaps 24 extend from all four planar sides (see Figure 4 (a)). In (b), the flaps are flattened while passing through tissue.

In the case of the catheter of the invention, the planar surface is integral to the position of the perforations and the two occur deliberately at the same point on the catheter surface. The planar surface allows normal functioning of the perforation flaps. It allows the flaps to move forward or backward without significant resistance and without noticeable distortion at the base of the aperture flap that would otherwise occur if the surface were non-planar. It is critical to the design of the present catheter that distortion not occur as this will potentially lead to unnecessary damage to normal brain tissue during insertion or removal of the catheter.

The straight fold line of the closure flaps in the catheter of the present invention is parallel to the planar surface portion on which they are located. This allows the flap to flap completely forward during insertion of the catheter into the brain to access the brain cavity, or completely backward during removal. It is imperative that the flaps fold line is such that the flaps move to parallel alignment with the direction of movement of the catheter during either insertion or removal. This prevents the cheese grater effect, which is unique to brain tissue, and consequent damage to adjacent delicate brain tissue.

A fold along the base of the radial flap adjacent the line of confluence with the planar surface, allows the flap to move both forward and backward with equal efficiency, and also allows the flap mechanics to prevent damage to the normal brain tissue adjacent the path of the catheter. No invention to date has identified this arrangement to achieve the two most desirable functions of a catheter for draining brain fluid.

Having the flaps stand proud from the body when the catheter is located in the brain cavities allows brain fluid to drain through the perforations. The proud flaps also prevent the catheter perforations from being occluded by lying up against the walls of the brain cavities. The invention allows the flaps to completely occlude the perforations during insertion and removal of the catheter without damaging normal brain tissue lying adjacent the passage of the catheter.

These embodiments merely illustrate non-limiting examples of the catheter device according to the invention. With the insight gained from this disclosure, the person skilled in the art is well placed to discern further realisations by means of which to put the claimed invention into practice.

## Claims

1. A catheter (10) for removing cerebrospinal fluid from the brain, the catheter (10) comprising:
an elongate body (12) having:
an internal lumen (14);
an exterior surface (18);
a perforation (22) that extends through the exterior surface, providing fluid communication between the lumen and said surface portion; and,
a movable covering operatively connected to the body (12) to occlude said perforation in response to relative movement between the catheter and tissue during insertion and withdrawal from said tissue,
***characterised in that** the* exterior surface (18) includes a portion (20) through which the perforation extends, the portion being substantially planar.

2. The catheter according to claim 1, wherein the covering stands proud from the body when the catheter is operatively located in tissue, thereby uncovering the perforation.

3. The catheter according to claim 2, wherein the covering is foldable to occlude the perforation.

4. The catheter according to claim 2 or 3, wherein the covering is foldable in opposite directions.

5. The catheter according to claim any one of the preceding claims, wherein the perforation is associated with two coverings.

6. The catheter according to any one of the preceding claims, wherein the covering comprises a flap (24).

7. The catheter according to claim 6, comprising a fold line (36) along which the flap is foldable to occlude the perforation.

8. The catheter according to claim 6 or 7, wherein the flap (24) is resiliently flexible.

9. The catheter according to any one of the preceding claims, wherein the covering and body are integral with each other.

10. The catheter according to any one of the preceding claims, wherein the lumen has a profile corresponding in shape to the axial profile of the body.

11. The catheter according to any one of the preceding claims, wherein the body comprises interconnected distal and proximal portions, having different respective axial profiles.

12. The catheter according to claim 11, wherein the distal portion includes the perforation.

13. The catheter according to claim 11 or claim 12, wherein the distal portion has a generally rectangular axial profile.

## Patentansprüche

1. Katheter (10) zum Entfernen von Gehirn-Rückenmarkflüssigkeit vom Gehirn, wobei der Katheter (10) umfasst:
einen länglichen Körper (12) mit:
einem internen Lumen (14);
eine Außenfläche (18);
eine Perforierung (22), die sich durch die Außenfläche erstreckt und eine fließende Verbindung zwischen dem Lumen und der genannten Außenfläche bereitstellt; und
eine bewegliche Abdeckung, die an den Körper (12) angeschlossen ist, um die genannte Perforierung als Reaktion auf die relative Bewegung zwischen dem Katheter und dem Gewebe während des Einfügens in das genannte Gewebe und der Abnahme daraus zu verschließen,
***dadurch gekennzeichnet, dass*** die Außenfläche (18) einen Abschnitt (20) enthält, durch den sich die Perforierung erstreckt, wobei der Abschnitt im Wesentlichen eben ist.

2. Katheter gemäß Anspruch 1, wobei die Abdeckung aus dem Körper hervorsteht, wenn der Katheter funktionsfähig in dem Gewebe angeordnet ist und dadurch die Perforierung freilegt.

3. Katheter gemäß Anspruch 2, wobei die Abdeckung faltbar ist, um die Perforierung zu verschließen.

4. Katheter gemäß Anspruch 2 oder 3, wobei die Abdeckung in entgegengesetzten Richtungen faltbar ist.

5. Katheter gemäß irgendeinem der voranstehenden Ansprüche, wobei die Perforierung zwei Abdeckungen zugeordnet ist.

6. Verfahren gemäß irgendeinem der voranstehenden Ansprüche, wobei die Abdeckung eine Klappe (24) umfasst.

7. Katheter gemäß Anspruch 6, umfassend eine Faltlinie (36), entlang derer die Klappe zum Verschließen der Perforierung faltbar ist.

8. Katheter gemäß Anspruch 6 oder 7, wobei die Klappe (24) elastisch flexibel ist.

9. Katheter gemäß irgendeinem der voranstehenden Ansprüche, wobei die Abdeckung und der Körper miteinander ein einziges Stück bilden.

10. Katheter gemäß irgendeinem der voranstehenden Ansprüche, wobei das Lumen ein Profil aufweist, das in der Form dem axialen Profil des Körpers entspricht.

11. Katheter gemäß irgendeinem der voranstehenden Ansprüche, wobei der Körper miteinander verbundene distale und proximale Abschnitte umfasst, die jeweilige unterschiedliche axiale Profile aufweisen.

12. Katheter gemäß Anspruch 11, wobei der distale Abschnitt die Perforierung enthält.

13. Katheter gemäß Anspruch 11 oder Anspruch 12, wobei der distale Abschnitt ein allgemein rechteckiges axiales Profil aufweist.

## Revendications

1. Cathéter (10) pour retirer du liquide cérébrospinal du cerveau, le cathéter (10) comprenant :
un corps allongé (12) ayant :
un lumen interne (14) ;
une surface extérieure (18) ;
une perforation (22) qui s'étend à travers la surface extérieure, pour fournir une communication fluidique entre le lumen et ladite surface extérieure; et,
un couvercle mobile relié fonctionnellement avec le corps (12) pour occulter ladite perforation en réponse au mouvement relatif entre le cathéter et le tissu pendant l'insertion et le retrait dudit tissu,
***caractérisé en ce que*** la surface extérieure (18) inclut une partie (20) à travers laquelle s'étend la perforation, la partie étant sensiblement plane.

2. Cathéter selon la revendication 1, dans lequel le couvercle fait saillie du corps lorsque le cathéter est disposé pour fonctionner dans le tissu, découvrant de ce fait la perforation.

3. Cathéter selon la revendication 2, dans lequel le couvercle est repliable pour occulter la perforation.

4. Cathéter selon la revendication 2 ou 3, dans lequel le couvercle est repliable dans des directions opposées.

5. Cathéter selon l'une quelconque des revendications précédentes, dans lequel la perforation est associée à deux couvercles.

6. Cathéter selon l'une quelconque des revendications précédentes, dans lequel le couvercle comprend un volet (24).

7. Cathéter selon la revendication 6, comprenant une ligne de pliage (36) le long de laquelle le volet est repliable pour occulter la perforation.

8. Cathéter selon la revendication 6 ou 7, dans lequel le volet (24) est élastiquement déformable.

9. Cathéter selon l'une quelconque des revendications précédentes, dans lequel le couvercle et le corps sont solidaires.

10. Cathéter selon l'une quelconque des revendications précédentes, dans lequel le lumen a un profil correspondant en forme au profil axial du corps.

11. Cathéter selon l'une quelconque des revendications précédentes, dans lequel le corps comprend des parties distale et proximale interconnectées, ayant des profils axiaux respectifs différents.

12. Cathéter selon la revendication 11, dans lequel la partie distale inclut la perforation.

13. Cathéter selon la revendication 11 ou 12, dans lequel la partie distale a un profil axial généralement rectangulaire.
